Europäisches Patentamt

European Patent Office

Office européen des brevets

(11) Veröffentlichungsnummer: **0 186 076**
**B1**

(12) **EUROPÄISCHE PATENTSCHRIFT**

(45) Veröffentlichungstag der Patentschrift: **26.09.90**

(51) Int. Cl.⁵: **C 07 C 69/28, C 07 C 67/44**

(21) Anmeldenummer: **85115957.4**

(22) Anmeldetag: **13.12.85**

(54) Verfahren zur Herstellung von 2,2,4-Trimethyl-1,3-pentandiolmonoisobutyrat.

(30) Priorität: **22.12.84 DE 3447029**

(43) Veröffentlichungstag der Anmeldung:
**02.07.86 Patentblatt 86/27**

(45) Bekanntmachung des Hinweises auf die
Patenterteilung:
**26.09.90 Patentblatt 90/39**

(84) Benannte Vertragsstaaten:
**AT BE CH DE FR GB IT LI LU NL SE**

(56) Entgegenhaltungen:
**DE-A-3 024 496**
**US-A-3 718 689**

(73) Patentinhaber: **HOECHST
AKTIENGESELLSCHAFT
Postfach 80 03 20
D-6230 Frankfurt am Main 80 (DE)**

(72) Erfinder: **Weber, Jügen, Dr. Dipl.-Chem.
Bunsenstrasse 17
D-4200 Oberhausen 11 (DE)**
Erfinder: **Hahn, Heinz-Dieter, Dr. Dipl.-Chem.
Burgstrasse 21
D-4200 Oberhausen 11 (DE)**
Erfinder: **Lappe, Peter, Dr. Dipl.-Chem.
Zum Ravenhorst 9
D-4200 Oberhausen 11 (DE)**
Erfinder: **Thönnessen, Franz, Dr. Dipl.-Chem.
Lützowstrasse 49
D-4200 Oberhausen 11 (DE)**
Erfinder: **Springer, Helmut, Dipl.-Ing.
Drostenkampstrasse 24
D-4200 Oberhausen 11 (DE)**

**Beschreibung**

Die vorliegende Erfindung betrifft ein Verfahren zur Herstellung von 2,2,4-Trimethyl-1,3-pentandiolmonoisobutyrat aus Isobutyraldehyd.

2,2,4-Trimethyl-1,3-pentandiolmonoisobutyrat ist ein ausgezeichnetes Verlaufshilfsmittel für Klebstoffe und Lacke auf Polyvinylacetat- und polyacrylat-Basis. Weiterhin wird es als Ausgangsstoff für die Herstellung von Farbstoffen, Weichmachern, Schmiermitteln, Lösungsmitteln und Herbiziden eingesetzt. Gegenüber anderen hochsiedenden Estern ist seine gute Hydrolysebeständigkeit hervorzuheben.

Der bei der Umsetzung von Isobutyraldehyd mit Natriumalkoholat als Katalysator entstehende Monoester wird in der Literatur zumeist als 2,2,4-Trimethyl-1,3-pentandiol-1-monoisobutyrat beschrieben. Neuere Untersuchungen haben jedoch gezeigt, daß das Reaktionsprodukt ein Gemisch der beiden isomeren Ester 2,2,4-Trimethyl-1,3-pentandiol-1-monoisobutyrat und 2,2,4-Trimethyl-1,3-pentandiol-3-monoisobutyrat ist (vgl. Liebigs Ann. Chem. 756, 162 bis 169, 1972).

Die kontinuierliche Herstellung von 2,2,4-Trimethyl-1,3-pentandiolmonoisobutyrat durch ein homogen-katalysiertes Verfahren ist Gegenstand der US-PS 3 718 689. Als Katalysator können wässrige Alkali- oder Erdalkalihydroxidlösungen eingesetzt werden. Da die Konzentration des Hydroxids in der wässrigen Lösung mindestens 30 Gew.% betragen soll, kommen wegen ihrer geringen Löslichkeit Erdalkalihydroxide als Katalysatoren kaum in Betracht. In den Beispielen werden dementsprechend fast ausschließlich 50 %ige wässrige Natriumhydroxidlösungen eingesetzt.

Wichtig für diese Arbeitsweise ist eine sehr intensive Durchmischung des Reaktionsgemisches. Nicht umgesetzter Isobutyraldehyd liegt nach der Reaktion als Isobutyraldoxan (2,6-Diisopropyl-5,5-dimethyl-1,3-dioxan-4-ol) vor und wird mit Wasserdampf bei 125°C und 0,2 MPa vollständig in den monomeren Isobutyraldehyd gespalten.

Diese Reaktionsführung erfordert unter Druck arbeitende Abtriebskolonnen, die störanfällig sind und die Wirtschaftlichkeit des Verfahrens beeinträchtigen. Zudem deuten die Ergebnisse der Beispiele 4 bis 6 darauf hin, daß die Umsetzung sehr empfindlich und nur schlecht reproduzierbar ist. Denn unter übereinstimmenden Reaktionsbedingungen werden in Beispiel 4 61 Gew.%, in Beispiel 5 45 Gew.% und in Beispiel 6 29 Gew.% 2,2,4-Trimethyl-1,3-pentandiolmonoisobutyrat gebildet, Der Anteil an nicht umgesetztem Isobutyraldehyd steigt von 36 Gew.% in Beispiel 4 über 51 Gew.% in Beispiel 5 auf 70 Gew.% in Beispiel 6 an. Bei Einsatz von Natronlauge, die nur 10 Gew.% NaOH enthält, reagiert der Isobutyraldehyd nicht mehr. Außer in homogener Phase mit Alkalihydroxidlösungen, die mehr als etwa 40 Gew.% Alkalihydroxid enthalten, kann die Umsetzung von Isobutyraldehyd auch in einem Zweiphasensystem - organische Phase, wässrige Phase- erfolgen.

Auf die Bedeutung einer gründlichen Durchmischung von Isobutyraldehyd und Katalysatorlösung, die als getrennte Phasen vorliegen, für die Umsetzung, wird auch in der US-PS 3 291 821 hingewiesen (Spalte 1, Zeilen 52—54).

Nach dem Verfahren der DE-OS 30 24 496 leitet man zur Herstellung von 2,2,4-Trimethyl-1,3-pentandiolmonoisobutyrat Isobutyraldehyd und wässrige Alkalihydroxidlösung gleichzeitig in ein Reaktionsgefäß. Nach Beendigung der Reaktion setzt man Wasser zu und destilliert den nicht umgesetzten Isobutyraldehyd als Azeotrop mit Wasser ab, wobei möglicherweise gebildetes Isobutyraldoxan in Isobutyraldehyd gespalten wird. Die separate Abtrennung des Isobutyraldehyds ist mit zusätzlichem Destillationsaufwand verbunden und beeinträchtigt dadurch die Wirtschaftlichkeit des Verfahrens.

Der in der US-PS 3 703 541 beschriebene Prozeß verwendet Alkaliphenolate als Katalysatoren für die Herstellung von 2,2,4-Trimethyl-1,3-pentandiol-1-monoisobutyrat. Umsatz und Selektivität nehmen bei diesem Verfahren mit steigendem Wassergehalt des Isobutyraldehyds ab. Daher wird gefordert daß der Aldehyd weniger als etwa 5500 ppm Wasser enthält. Diese Arbeitsweise ist, bedingt durch den Einsatz von Alkaliphenolaten und die notwendige Entwässerung des Isobutyraldehyds, sowohl technisch als auch wirtschaftlich sehr aufwendig.

Aus der DE-OS 2 820 518 ist es bekannt, 2,2,4-Trimethyl-1,3-pentandiolmonoisobutyrat durch Reaktion von Isobutyraldehyd in Gegenwart von Erdalkalihydroxid und Wasser und unter Zusatz von Carbonsäure, in Form der freien Säure, ihres Alkalisalzes und/oder ihres Erdalkalisalzes herzustellen. Nachteile dieses Verfahrens sind, daß das Reaktionsgemisch nach Beendigung der Umsetzung mit Kohlendioxid neutralisiert werden muß und daß sich die Calciumsalze aus dem Reaktionsgemisch sehr schlecht abtrennen lassen. Die bekannten Verfahren erfordern entweder den Einsatz hochkonzentrierter Alkalihydroxidlösungen, um die Reaktion in homogener Phase durchzuführen oder eine sehr intensive Durchmischung der ineinander kaum löslichen wässrigen und organischen Phase.

Es bestand daher die Aufgabe, ein Verfahren zur Herstellung von 2,2,4-Trimethyl-1,3-pentandiolmonoisobutyrat zu entwickeln, das die aufgezeigten Mängel des Standes der Technik vermeidet und das bei einfacher Reaktionsführung die Gewinnung des reinen Monoesters in hohen Selektivitäten ermöglicht.

Die Erfindung besteht in einem Verfahren zur Herstellung von 2,2,4-Trimethyl-1,3-pentandiolmonoisobutyrat aus Isobutyraldehyd in Gegenwart wässriger Alkalihydroxidlösungen als Katalysator in einem Rohrreaktor. Es ist dadurch gekennzeichnet, daß als Alkalihydroxidlösung wässrige Natronlauge mit einem Natriumhydroxidgehalt von 20 bis 30 Gew.-% eingesetzt wird.

2

Überraschenderweise gestattet das erfindungsgemäße Verfahren bei einfachster Reaktionsführung Isobutyraldehyd mit hoher Selektivität in 2,2,4-Trimethyl-1,3-pentandiolmonoisobutyrat zu überführen.

Nach einer bevorzugten Ausführungsform der neuen Arbeitsweise leitet man gleichzeitig Isobutyraldehyd unmittelbar und die Katalysatorlösung über ein Tauchrohr in den Rohrreaktor.

Als Katalysatorlösung wird erfindungsgemäß Natronlauge, die Natriumhydroxid in einer Konzentration von 20 bis 30 Gew.-% enthält, verwendet. Je Mol Isobutyraldehyd setzt man 10 bis 100 mmol Alkalihydroxid ein. An die Reinheit des Isobutyraldehyds werden keine besonderen Anforderungen gestellt. Ein Vorteil des Verfahrens ist die Möglichkeit, Wasser enthaltenden Isobutyraldehyd einzusetzen, der technisch leicht zugänglich ist.

Die Umsetzung der Reaktanten erfolgt bei Temperaturen von 50 bis 80°C. Die Verweilzeit ist von der jeweiligen Arbeitstemperatur abhängig, sie beträgt 0,25 bis 2 Stunden, insbesondere 0,4 bis 1 Stunde.

Überraschenderweise wird unter sonst konstanten Reaktionsbedingungen durch eine Verkürzung der Reaktionszeit die 2,2,4-Trimethyl-1,3-pentandiolmonoisobutyrat-Ausbeute bei gleichzeitigem Vorhandensein nicht umgesetzten Isobutyraldehyds erhöht.

Dieses Ergebnis war nicht zu erwarten, da üblicherweise bei chemischen Umsetzungen die Ausbeute durch eine Verkürzung der Reaktionszeit — bei gleichzeitigem Vorhandensein von Ausgangsstoffen — abnimmt.

Nach einer bevorzugten Variante des erfindungsgemäßen Prozesses führt man die Umsetzung in einem mit Füllkörpern gefüllten Rohrreaktor durch. Geeignet sind z.B. Raschig-Ringe. Das am Kopf des Reaktors übergehende Reaktionsgemisch wird in einem Phasentrenner in die organische und wässrige Phase getrennt. Die organische Phase kann ohne weitere Reinigung in den nicht umgesetzten Isobutyraldehyd und den Monoester getrennt werden. Der nicht umgesetzte Isobutyraldehyd kann ohne weitere Reinigung in den Reaktor zurückgeführt werden.

Das erfindungsgemäße Verfahren wird kontinuierlich durchgeführt, es zeichnet sich gegenüber dem üblichen Herstellverfahren im Rührkessel dadurch aus, daß der apparative Aufwand sowohl für die eigentliche Reaktion wie auch für die Aufarbeitung des Reaktionsgemisches wesentlich geringer ist.

Beispiel 1

Die Reaktion wird in einem senkrecht stehenden Doppelmantelrohr durchgeführt, das einen inneren Durchmesser von 36 mm und eine Länge von 120 cm aufweist und am unteren Ende eine Glasfritte G3 als Verteilerorgan besitzt. Das Rohr hat ein effektiv genutztes Reaktionsvolumen von 1220 ml und ist mit einer äquimolaren Mischung aus Isobuttersäure und 20 %iger Natronlauge gefüllt. Bei einer Temperatur von 72°C werden stündlich 1350 ml Isobutyraldehyd durch die Glasfritte in den Reaktor geleitet. Vom oberen Ende des Reaktors werden durch ein Einleitrohr, das 20 cm oberhalb der Glasfritte endet, 118 ml 20 %iger Natronlauge/h zugegeben. Das organische Reaktionsprodukt und die verbrauchte Katalysatorlösung werden am oberen Ende des Reaktors durch einen als Überlauf angeordneten Kühler abgenommen. Bei konstantem Reaktorvolumen beträgt die Gesamtdurchsatzmenge 1468 ml entsprechend einer Verweilzeit von 0,83 h.

Die organische Phase (1037 g/h) wird bei Normaldruck in geeigneter Weise bis 160°C Sumpftemperatur andestilliert, wobei als Destillat nicht umgesetzter Isobutyraldehyd in direkt wiedereinsetzbarer Form anfällt. Der Rückstand (38,2 % vom Einsatz) besteht nach gaschromatographischer Analyse aus

3,4 % Isobutyraldehyd
19,8 % 2,2,4-Trimethyl-1,3-pentandiol
75,5 % 2,2,4-Trimethyl-1,3-pentandiolmonoisobutyrat
1,3 % Sonstige Komponenten

Durch weitere Destillation erhält — man bezogen auf 1000 g organisches Rohprodukt nach Reaktion — 281 g 99,8 %igen Monoester.

Beispiel 2

Die in Beispiel 1 beschriebene Apparatur wird mit 5 mm Glasringen gefüllt. Bei einer Temperatur von 71°C werden stündlich 2100 ml Isobutyraldehyd und 180 ml 20 %ige Natronlauge zugeführt. Die Gesamtdurchsatzmenge beträgt 2280 ml entsprechend einer Verweilzeit von 0,54 h. Der organische Anteil (1593 g/h) der abgenommenen flüssigen Phasen wird wie oben beschrieben von nicht umgesetztem Isobutyraldehyd befreit. Der Rückstand (49,6 % von Einsatz) besteht nach gaschromatographischer Analyse aus

6,5 % Isobutyraldehyd
15,3 % 2,2,4-Trimethyl-1,3-pentandiol
74,8 % 2,2,4-Trimethyl-1,3-pentandiolmonoisobutyrat
3,4 % Sonstige Komponenten

Durch weitere Destillation erhält man — bezogen auf 1000 g organische Phase nach Reaktion — 365 g 99,8 %igen Monoester.

## Beispiel 3

Die in Beispiel 1 beschriebene Apparatur wird mit 5 mm Glasringen gefüllt. Bei einer Temperatur von 64°C werden stündlich 1325 ml Isobutyraldehyd und 90 ml 20 %ige Natronlauge zugeführt. Die Gesamtdurchsatzmenge beträgt 1415 ml entsprechend einer Verweilzeit von 0,86 h. Der organische Anteil (983 g/h) der abgenommenen flüssigen Phasen wird, wie in Beispiel 1 beschrieben, vom nicht umgesetzten Isobutyraldehyd befreit. Der Rückstand (34,3 % vom Einsatz) besteht nach gaschromatographischer Analyse aus

7,6 % Isobutyraldehyd
14,0 % 2,2,4-Trimethyl-1,3-pentandiol
76,1 % 2,2,4-Trimethyl-1,3-pentandiolmonoisobutyrat
2,3 % Sonstige Komponenten

Durch weitere Destillation erhält man — bezogen auf 1000 g organische Phase nach Reaktion — 257 g 99,8 %igen Monoester.

## Beispiel 4

Es wird analog Beispiel 3 verfahren: Bei einer Temperatur von 64°C werden dem Reaktor stündlich 2725 ml Isobutyraldehyd und 185 ml 20 %ige Natronlauge zugeführt. Die Gesamtdurchsatzmenge beträgt 2910 ml entsprechend einer Verweilzeit von 0,42 h. Die organische Phase (2103 g/h) wird, wie in Beispiel 1 beschrieben, vom nicht umgesetzten Isobutyraldehyd befreit. Der Rückstand (44,8 % vom Einsatz) besteht nach gaschromatographischer Analyse aus

6,5 % Isobutyraldehyd
11,4 % 2,2,4-Trimethyl-1,3-pentandiol
79,9 % 2.2.4-Trimethyl-1,3-pentandiolmonoisobutyrat
2,2 % Sonstige Komponenten

Durch weitere Destillation erhält man 8 bezogen auf 1000 g organisches Rohprodukt nach Reaktion — 352 g 99,8 %igen Monoester.

## Patentansprüche

1. Verfahren zur Herstellung von 2,2,4-Trimethyl-1,3-pentandiolmonoisobutyrat aus Isobutyraldehyd in Gegenwart wäßriger Alkalihydroxidlösungen als Katalysator in einem Rohrreaktor, dadurch gekennzeichnet, daß als Alkalihydroxidlösung wäßrige Natronlauge mit einem Natriumhydroxidgehalt von 20 bis 30 Gew.-% eingesetzt wird.

2. Verfahren nach Anspruch 1, dadurch gekennzeichnet, daß der Rohrreaktor Füllkörper enthält.

3. Verfahren nach Anspruch 1 und 2, dadurch gekennzeichnet, daß man gleichzeitig Isobutyraldehyd unmittelbar und die Katalysatorlösung über ein Tauchrohr in den Rohrreaktor leitet.

4. Verfahren nach Anspruch 1 bis 3, dadurch gekennzeichnet, daß die Umsetzung der Reaktanten bei Temperaturen von 50 bis 80°C erfolgt.

5. Verfahren nach Anspruch 1 bis 4, dadurch gekennzeichnet, daß die Verweilzeit der Reaktanten im Reaktor 0,25 bis 2 Stunden beträgt.

## Revendications

1. Procédé pour la fabrication de monoisobutyrate de 2,2,4-triméthyl-1,3-pentanediol à partir d'isobutyraldéhyde en présence de solutions aqueuses d'hydroxydes alcalins comme catalyseurs dans un réacteur tubulaire, caractérisé en ce que l'on utilise comme solution d'hydroxyde alcalin une lessive de soude aqueuse ayant une teneur en hydroxyde de sodium de 20 à 30 % en poids.

2. procédé selon la revendication 1, caractérisé en ce que le réacteur tubulaire contient des corps de garnissage.

3. Procédé selon les revendications 1 et 2, caractérisé en ce qu'on envoie simultanément dans le réacteur tubulaire l'isobutyraldéhyde directement et la solution de catalyseur au moyen d'un tube plongeant.

4. Procédé selon les revendications 1 à 3, caractérisé en ce que la réaction des réactifs s'effectue à des températures de 50 à 80°C.

5. Procédé selon les revendications 1 à 4 caractérisé en ce que la durée de passage des réactifs dans le réacteur est de 0,25 à 2 h.

**Claims**

1. A process for preparing 2,2,4-trimethyl-1,3-pentane diolmonoisobutyrate from isobutyraldehyde in the presence of aqueous alkali metal hydroxide solutions as catalysts in a tubular reactor, characterised in that aqueous sodium hydroxide solution with a sodium hydroxide content of 20 to 30% by weight is used as the alkali metal hydroxide solution.

2. A process according to claim 1, characterised in that the tubular reactor contains packing material.

3. A process according to claims 1 and 2, characterised in that isobutyraldehyde and the catalyst solution are fed simultaneously into the reactor, the former directly and the latter via a dip pipe.

4. A process according to claims 1 to 3, characterised in that the reactants are reacted at temperatures of 50 to 80°C.

5. A process according to claims 1 to 4, characterised in that the residence period of the reactants in the reactor is 0.25 to 2 hours.